# EUROPEAN PATENT APPLICATION

(11) **EP 4 636 075 A1**
(43) Date of publication of application: **22.10.2025**
(21) Application number: 23903710.4
(22) Date of filing: 16.10.2023
(51) Int. Cl.: C12N 5/00

(54) **METHOD AND DEVICE FOR THREE-DIMENSIONAL LEVITATION CULTURE OF ORGANOIDS**

(30) Priority: 14.12.2022 KR 20220174402; 12.10.2023 KR 20230135652
(71) Applicant: Radahaim Co., Ltd., Seongnam-si, Gyeonggi-do 13466 (KR)
(72) Inventor: LEE, Heon Ju, Yongin-si Gyeonggi-do 17009 (KR); AN, Jee Young, Seoul 03066 (KR); LIM, Hyun Wook, Seoul 04726 (KR)
(74) Representative: Hautier IP - MC/EP
(86) International application number: PCT/KR2023/015960
(87) International publication number: WO 2024/128506

(57) **Abstract**

Disclosed in the present disclosure is a method for three-dimensional levitation culture of organoids, comprising the steps of: injecting a fluid including organoid seeds, which include magnetic nanoparticles, into a microchannel of a microfluidic chip; providing magnetic force formed by means of a magnet to the organoid seeds injected into the microchannel, so that the organoid seeds levitate at an incubation position; holding the organoid seeds in the incubation position by means of the magnetic force; (d) culturing the organoid seeds into an organoid while replacing a culture medium in the microchannel; and collecting the cultured organoids.

## Description

### [Technical Field]

The present disclosure relates to the preparation of organoids, and more particularly, to a method and device for three-dimensional levitation culture of organoids by levitating organoid seeds in a culture medium and culturing organoids in a three-dimensional manner.

### [Background Art]

In a process of developing new drugs, animal experiments on animals similar to humans are required prior to conducting clinical trials. Animals such as rats and rabbits are used in animal experiments, and the ethics therefor are questionable. In addition, since animal experimental models have different genetic or biological characteristics from humans, drug tests using existing animal experimental models have limitations in the reliability of drug test reactions, such as side effects that have not been found in animal experiments, but are found in humans.

Moreover, recently, as the US FDA removed the mandatory provision of requiring animal experiments as a condition for new drug approval, various methods for new preclinical test models for new drug development have been studied.

However, conventional 2D cell line culture methods have the disadvantage of being difficult to reproduce the unique properties and characteristics of tissues, and cancer patient-derived xenograft models are also used as an alternative, but have the disadvantage of being unsuitable for large-scale drug screening.

Therefore, organoids have recently attracted significant attention as a new preclinical test model for new drug development.

The organoids are organoids prepared by culturing or recombining stem cells in a three-dimensional manner, and are derived from various organs in the body and contain stem cells therein to form aggregates that may differentiate into cell bodies. In addition, organoids derived from each organ in the body have a structure very similar to the corresponding organ, and thus may be used as models capable of replacing cell and animal experiments.

However, conventionally cultured organoids had limitations in use in actual new drug development due to problems with consistency and reproducibility of study results. This is because cell culture varies depending on a laboratory environment, and the size and degree of differentiation vary depending on a batch even when cultured under the same conditions, so that there are limitations to obtain statistically significant data.

To solve the problems in the related art, an organoid culture technology is required so that cultured organoids may be produced in a homogeneous form under certain conditions even while enabling mass production of organoids.

Meanwhile, 3D culture technology is required to produce organoids into human-like tissues or mini-organs. However, it is almost impossible to perfectly culture organoids in a three-dimensional manner. Therefore, a 3D culture method has been the most widely used to form a Matrigel dome and grow stem cells on the Matrigel dome. However, strictly speaking, since the organoids are not cultured in a three-dimensional manner, but rather grow in a form where a part comes into contact with the bottom, it was very difficult to differentiate into organoids similar to human tissues or organs consisting of various cells. Many researchers studying organoids also point out the reason why organoids are still unable to 100% mimic human organs. To overcome the problem, NASA has conducted research to culture stem cells in space without gravity.

### [Disclosure]

### [Technical Problem]

An object of the present disclosure is to provide a method and device for three-dimensional levitation culture of organoids, which levitates organoid seeds in a microchannel filled with a culture medium and cultures organoids in a three-dimensional manner while replacing the culture medium.

### [Technical Solution]

According to an aspect of the present disclosure, a method for three-dimensional levitation culture of organoids includes a) injecting a fluid including organoid seeds, which include magnetic nanoparticles, into a microchannel of a microfluidic chip; b) providing magnetic force formed by means of a magnet to the organoid seeds injected into the microchannel, so that the organoid seeds levitate at a culture position; c) holding the organoid seeds in the culture position by means of the magnetic force; d) culturing the organoid seeds into an organoid while replacing a culture medium in the microchannel; and e) collecting the cultured organoids.

According to one aspect of the present disclosure, the organoid seeds may include stem cells, growth factors, biodegradable materials, and may include an extracellular matrix.

According to one aspect of the present disclosure, the organoid seeds are in a gelled state.

According to one aspect of the present disclosure, the method may include an alignment step of aligning the organoid seeds at positions set to be spaced apart from each other before step b), respectively, and the alignment step may be performed by capturing the organoid seeds within alignment grooves formed in the microchannel while blocking the inflow and outflow of the fluid in the microchannel.

According to one aspect of the present disclosure, the alignment grooves are formed to be continuously connected to each other along the inner side of the microchannel so that the connected form of the alignment grooves has a wave shape, the alignment grooves are formed to have constant pitches, the organoid seeds are injected so as to be spaced apart from each other in response to the pitches of the alignment grooves, and the organoid seeds are aligned by moving to the lower part along the inclined surface of the alignment grooves by gravity.

According to one aspect of the present disclosure, the alignment grooves are formed along the inner side of the microchannel, and the alignment step is performed by rotating the microfluidic chip so that the alignment grooves face downward.

According to one aspect of the present disclosure, the magnetic poles of the magnets facing each other with the microfluidic chip interposed therebetween have different polarities.

According to one aspect of the present disclosure, the magnetic poles of the adjacent magnets arranged on the same plane have different polarities.

According to another aspect of the present disclosure, a device for three-dimensional levitation culture of organoids includes: an inlet into which a fluid including organoid seeds, which include magnetic nanoparticles, and a culture medium are injected; a microchannel connected to the inlet and into which the organoid seeds are separately injected at predetermined injection intervals and moved, and held and cultured at a predetermined culture position; an outlet through which the culture medium and the cultured organoids are discharged; a microfluidic chip including valves capable of blocking the movement of the fluid to the inlet and the outlet; and a magnet array disposed on both opposite surfaces of the microfluidic chip, and including magnets providing magnetic force to levitate and hold the organoid seeds in the microchannel at the culture position.

According to another aspect of the present disclosure, alignment grooves are formed on the inner surface of the microchannel so that the organoid seeds are aligned by moving downward by gravity.

According to another aspect of the present disclosure, the alignment grooves are formed to be continuously connected to each other along the inner side so that the inner side has a wave shape, the alignment grooves are formed to have constant pitches, and the organoid seeds are injected to be spaced apart from each other in response to the pitches of the alignment grooves.

According to another aspect of the present disclosure, the magnet array includes a spacer having a plurality of magnet insertion grooves into which the magnets are inserted, and a plurality of magnets inserted into the magnet insertion grooves, and when the magnet arrays are arranged on both surfaces of the microfluidic chip, respectively, the magnetic poles of the magnets are aligned in response to the culture position of the organoid within the microchannel.

### [Advantageous Effects]

According to the method and device for three-dimensional levitation culture of organoids according to the present disclosure, organoid seeds are levitated in a culture medium and cultured into organoids while the culture medium is periodically replaced, so as to enable a three-dimensional culture in the practical sense.

According to the present disclosure, the organoid seeds grow under uniform conditions while the culture medium is periodically replaced within the microchannel, which is advantageous in culturing organoids with homogeneous characteristics. When the cultured organoids are sorted by size and provided for experiments for new drug development and the like, it is advantageous in ensuring consistency and reproducibility of research results.

The method of preparing organoids using the method and device for three-dimensional levitation culture of organoids according to the present disclosure can effectively perform mass production of organoids.

### [Description of Drawings]

FIG. 1 is a flow chart of a method for preparing organoids according to an embodiment of the present disclosure.
FIG. 2 is a flow chart of a method for preparing organoid seeds according to an embodiment of the present disclosure.
FIG. 3 is a flow chart of a three-dimensional levitation culture process of organoids according to the present disclosure.
FIG. 4 is a plan view of a microfluidic chip of a device for three-dimensional levitation culture of organoids according to an embodiment of the present disclosure, and FIG. 5 is a side view of the microfluidic chip illustrated in FIG. 4.
FIG. 6 is a diagram for describing an alignment process of organoid seeds in a device for three-dimensional levitation culture of organoids according to an embodiment of the present disclosure.
FIGS. 7 and 8 are diagrams for describing coupling of a microfluidic chip and a magnet array according to an embodiment of the present disclosure.
FIG. 9 illustrates a form in which a plurality of microfluidic chips are assembled in parallel in a device for three-dimensional levitation culture of organoids according to an embodiment of the present disclosure.
FIG. 10 is a diagram for sequentially describing coupling of a microfluidic chip and a magnetic array and a method for three-dimensional culture of organoids using the same.
FIG. 11 is a diagram showing a process of levitating and holding organoid seeds within a microchannel by magnetic force and culturing the organoids.
FIG. 12 is a fluorescence image diagram showing the survival rate of Hek cell seeds cultured in a 96-well plate.
FIG. 13 is a fluorescence image diagram showing the survival rate of Hek cell seeds cultured in a device for three-dimensional levitation culture according to an embodiment of the present disclosure.

### [Best Mode]

Hereinafter, embodiments of the present disclosure will be described with reference to the accompanying drawings.

The present disclosure may have various modifications and various embodiments, and specific embodiments will be illustrated in the drawings and described in detail in the detailed description. However, it should be understood that the present disclosure is not limited to specific embodiments, but covers all the modifications, equivalents and replacements included within the idea and technical scope of the present disclosure. In describing the present disclosure, a detailed description of known related arts will be omitted if it is determined that they make the gist of the present disclosure unclear.

In this specification, an "organoid seed" refers to a seed formed and cultured using an organoid seed composition to become an organoid, and a "composition for preparing organoids" is a cell composition including stem cells provided for culturing the organoids.

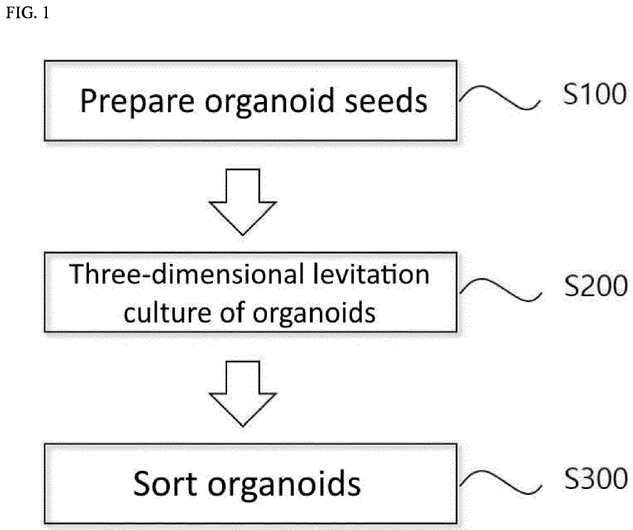

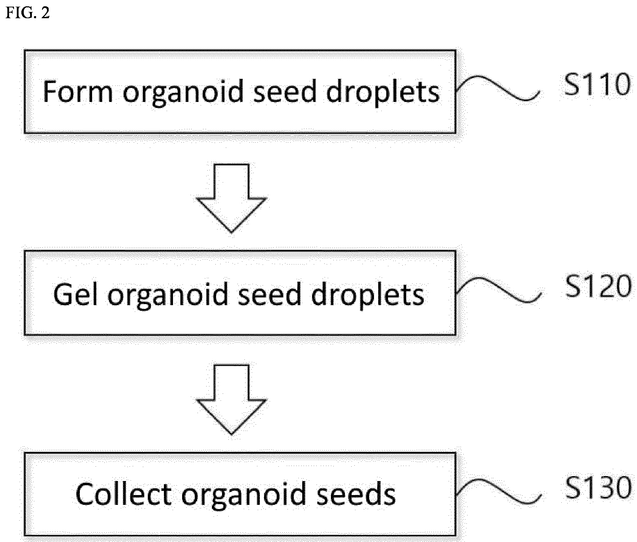

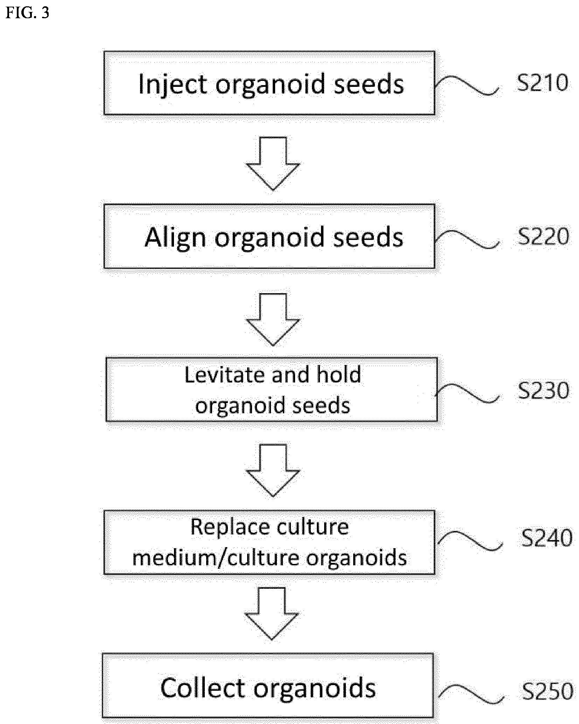

FIG. 1 is a flow chart of a method for preparing organoids according to an embodiment of the present disclosure, FIG. 2 is a flow chart of a method for preparing organoid seeds according to an embodiment of the present disclosure, and FIG. 3 is a flow chart of a method for three-dimensional levitation culture of organoids according to the present disclosure.

Referring to FIG. 1, a method for preparing organoids according to the present disclosure includes an organoid seed preparation step (S100), an organoid three-dimensional levitation culture step (S200), and an organoid sorting step (S300).

The organoid seed preparation step may be performed using a microfluidic chip for preparing organoid seeds. The organoid seed preparation step includes supplying an organoid seed composition into a microchannel of a microfluidic chip for preparing organoid seeds to continuously form organoid seed droplets in a form of levitating and moving in the solution (S110); gelling the organoid seed droplets (S120); and collecting the organoid seeds (S130). The organoid seeds are separately prepared continuously in the form of spheres levitated in the solution.

The gelling of the organoid seeds is not limited to be performed continuously after formation of the organoid seed droplets within the microfluidic chip, but may be collected from the microfluidic chip and the gelling may be performed in a separate device. For example, the organoid seeds may be gelled in the culture device for the organoid seeds.

The organoid seeds contain stem cells, biodegradable materials, growth factors, and extracellular matrixes.

Stem cells are precursor cells for organoid differentiation and may include adult stem cells (ASCs), embryonic stem cells (ESCs), and/or induced pluripotent stem cells (iPSCs). For example, the stem cells may be primary stem cells, proliferated stem cells, or partially differentiated stem cells. Specifically, the stem cells may be primary cells, which may be obtained directly from living tissue. In addition, the stem cells may be secondary cells, which may be cultured and/or subcultured cells.

As the biodegradable materials, gelatin, gelatin methacrylate (GelMA), collagen, poly(ethylene glycol) (PEG), Pluronic^{®}, alginate, and the like may be used. The biodegradable materials are contained for gelation of organoid seeds, and the gelation process varies depending on a type of biodegradable material.

For example, when the biodegradable material included in the organoid seed composition includes alginate, calcium chloride (CaCl₂) may be supplied as a gelling solution to the organoid seed droplets to cause gelation. In addition, for example, when the biodegradable materials included in the organoid seed composition are collagen and gelatin, the gelling solution may be a temperature-controlled medium or a saline solution. In addition, for example, if the biodegradable material included in the organoid seed composition is pluronic, the gelling solution may be a medium or a saline solution. In addition, for example, when the biodegradable material included in the organoid seed composition is fibrin, the gelling solution may be thrombin, and when the biodegradable material included in the organoid seed composition is thrombin, the gelling solution may be used with fibrin. The fibrin and thrombin react with each other to form fibrinogen drops. In addition, for example, when the organoid seed composition includes gelma as a biodegradable material, the organoid seed droplets may be gelled by UV irradiation.

The biodegradable materials maintain the shape of organoid seeds through gelation and may be used as components that construct tissues as cells grow. The biodegradable materials are gelled into a hydrogel state containing stem cells, growth factors, and extracellular matrix therein. The gelled biodegradable materials allow the exchange of oxygen and carbon dioxide and the absorption of nutrients supplied from the medium therein, and retain moisture. The biodegradable materials are degraded and then disappear as the stem cells grow and are cultured into organoids.

The extracellular matrix (ECM) may be a component included to differentiate the stem cells into organoids. The stem cells may differentiate into organoids by coming into contact with the extracellular matrix, and a type of extracellular matrix and the like may be controlled according to the characteristics of a desired organoid.

According to an embodiment of the present disclosure, the extracellular matrix may be obtained by culturing extracellular matrix-producing cells, such as epithelial cells, endothelial cells, exterior endoderm-like cells, or fibroblasts, and then removing the cells. The extracellular matrix-producing cells may be selected from chondrocytes which mainly produce collagen and proteoglycans; fibroblasts which mainly produce type IV collagen, laminin, interstitial procollagen and fibronectin; and colonic myofibroblasts which mainly produce collagen (types I, III and V), chondroitin sulfate proteoglycan, hyaluronic acid, fibronectin and tenascin-C. In addition, the extracellular matrix may be applied with commercially available extracellular matrixes such as Matrigel.

The growth factors are biologically active substances of the protein family that exist in the body and regulate cell growth, proliferation, and differentiation. As the growth factors, tens of types such as EGF, FGF, IGF, KGF, VEGF, and PDGF are known.

According to an embodiment of the present disclosure, the organoid seeds include magnetic nanoparticles. The magnetic nanoparticles may exhibit cytotoxicity, and are known to depend on a size and a concentration. According to an embodiment of the present disclosure, the magnetic nanoparticles are used at a diameter of 50 nm or less and a concentration of 50 µg/ml or less, and in this case, it was determined that there was no cytotoxicity. The magnetic nanoparticles are not limited thereto, and may be used in any size and concentration without cytotoxicity.

The magnetic nanoparticles may be included separately or in multiples in the organoid seeds.

The organoid seeds are transferred to the device for three-dimensional levitation culture of organoids, and the method for three-dimensional levitation culture of organoids is performed.

FIG. 3 is a flow chart of a method for three-dimensional levitation culture of organoids according to the present disclosure.

Referring to FIG. 3, the method may include an organoid seed injection step (S210), an organoid seed levitating and holding step (S230), an organoid culturing step (S240), and an organoid collection step (S250), and may include an alignment step (S220) for aligning organoid seeds before the organoid seed levitating and holding step.

The organoid seed injection step (S210) includes injecting the organoid seeds into a microchannel together with a fluid. As described above, the organoid seeds to be used contain magnetic nanoparticles. The fluid is intended to be injected into the microchannel without damaging the organoid seeds and is not limited by a specific component. The fluid may also be a saline solution or culture medium.

The organoid seeds are injected separately into the microchannel while mixed in the fluid.

The organoid alignment step (S220) is a step in which organoid seeds separately supplied to the microchannel are uniformly arranged along the microchannel while being spaced apart from each other. As the organoid seeds are arranged at a predetermined position within the microchannel, coupling of the magnet array with the microfluidic chip is advantageous to be levitated and held at the predetermined position by magnetic force without agglomeration of organoid seeds, etc. The organoid seeds are aligned using alignment grooves formed on the inner surface of the microchannel with gravity. To this end, the microfluidic chip may be rotated 90° (degrees) from a horizontal position where the organoid seeds are introduced to a vertical position.

The organoid seed levitating and holding step (S230) is performed by coupling the magnetic arrays to both surfaces of the microfluidic chip so that the organoid seeds are levitated in a direction opposite to gravity in the culture medium by magnetic force. Accordingly, the organoid seeds are pressed in a specific direction by the gravity so as to prevent the growth in the gravity direction from being hindered or inhibited, thereby enabling a practical three-dimensional culture.

The organoid culturing step (S240) is a step of culturing organoids while replacing the culture medium. Nutrients required for organoid growth are supplied by periodically replacing the culture medium to induce and grow differentiation of organoids.

The organoid collection step (S250) is a step of collecting the organoids from the microchannel to the outside. The magnet array is detached from the microfluidic chip to allow the organoid seeds to move along a fluid flow within the microchannel. The organoids are discharged through the outlet of the microfluidic chip together with the fluid and then collected.

Referring back to FIG. 1, the sorting step (S300) is performed by sorting the collected organoids by size. Since the organoids are cultured under uniform conditions in a three-dimensional levitation culture method, homogeneity of characteristics may be secured. By resorting these organoids by size to ensure homogeneity in size, it is easy to secure reproducibility of the experiment.

The organoid sorting step (S300) sorts organoids into several predetermined groups. The size range of organoids forming a group is appropriately selected by considering homogeneity.

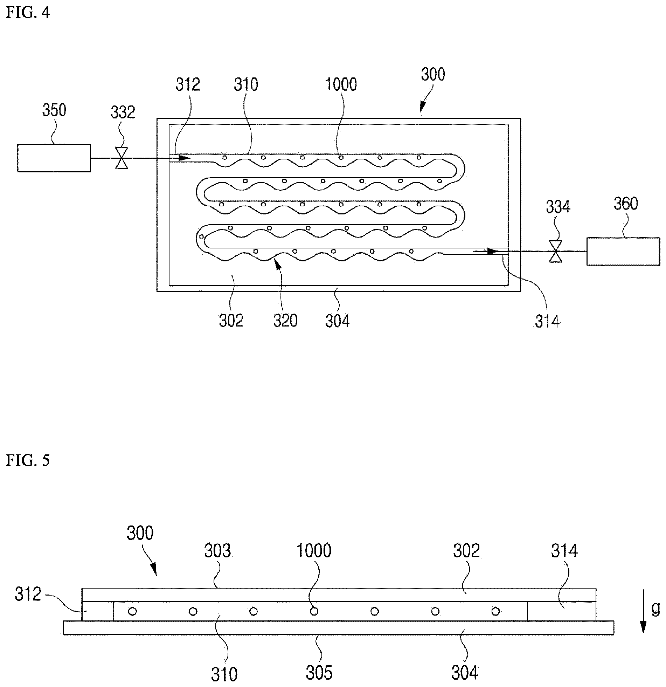

FIG. 4 is a plan view of a microfluidic chip of a device for three-dimensional levitation culture of organoids according to an embodiment of the present disclosure, and FIG. 5 is a side view of the microfluidic chip illustrated in FIG. 4.

The microfluidic chip 300 is manufactured as a flat plate. The microfluidic chip 300 is formed by bonding a first plate 302 and a second plate 304. The microfluidic chip 300 includes a microchannel 310 therein.

The first plate 302 and the second plate 304 may be manufactured from inorganic materials such as glass or silicon, and may be manufactured using polymer materials such as poly(dimethyl) siloxane (PDMS) or polymethylmethacrylate (PMMA).

The first plate 302 and the second plate 304 may be formed of different materials. According to an embodiment of the present disclosure, the second plate 304 is formed of a glass material, and the first plate 302 is made of a PDMS material.

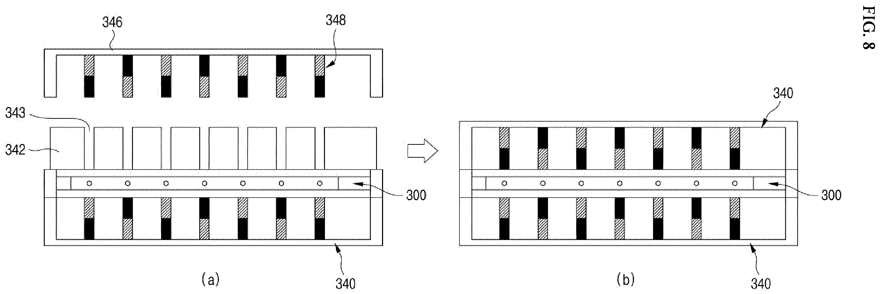

The microfluidic chip 300 has flat surfaces on both sides, i.e., a first surface 303 forming the upper surface of the first plate 302 and a second surface 305 forming the lower surface of the second plate 304, and while the microfluidic chip 300 is in an erected state, the magnet array 340 is coupled by contacting the first and second surfaces 303 and 305 (see FIG. 8).

A pattern forming the microchannel 310 is formed on the lower surface of the first plate 302, and the second plate 304 and the first plate 302 are bonded to each other. As a result, the microchannel 310 may be formed between the first plate 302 and the second plate 304.

As the length of the microchannel 310 is increased, the capacity to accommodate organoid seeds 1000 is improved. To increase the length of the microchannel 310, the microchannel 310 may be extended in a zigzag shape within the microfluidic chip 100.

The microchannel 310 is formed with a channel width through which the organoid seeds 1000 may be introduced, cultured, and then collected. According to an embodiment of the present disclosure, the microchannel may have the width and depth of approximately 2 to 6 mm.

The microchannel 310 has an inlet 312 and an outlet 314.

A fluid containing organoid seeds is injected into the microchannel 310 through the inlet 312. The fluid may be a culture medium, a saline solution, etc.

Through the outlet 314, the fluid such as a culture medium and a saline solution, and differentiated organoids derived from stem cells in the organoid seeds are discharged.

The inlet 312 may be connected with a supply unit 350. The supply unit 350 may be connected with the inlet 312 to inject the organoid seed 1000 into the microchannel 310 together with the fluid. In addition, the culture medium is injected through the inlet 312 to replace the culture medium.

The outlet 314 may be connected with a collection unit 360. The collection unit 360 may collect and discard the culture medium, the saline solution, etc., and may collect differentiated organoids to provide the collected organoids for the next process.

The microfluidic chip 300 may include valves 332 and 334 for controlling the movement of the fluid in the microchannel 310. Specifically, the microfluidic chip 300 includes a first valve 332 which blocks the movement of the fluid to the inlet 312 of the microfluidic chip 300 and a second valve 334 which blocks the movement of the fluid to the outlet 314.

When the movement of the fluid into the microchannel 310 is permitted, for example, in the cases of injecting the fluid containing organoid seeds, injecting and discharging the saline solution or the culture medium, or discharging the cultured organoids, the valves 332 and 334 are opened. In the case of arranging the organoid seeds 1000, culturing the organoid seeds 1000 in a culture medium, etc., the valves 332 and 334 are closed to isolate the microchannel 310 from the outside.

The valves 332 and 334 may be formed with micro valves, etc., and may be formed integrally with the microfluidic chip 300, or may be formed in the conduits connected to the inlet 312 and the outlet 314, respectively.

According to an embodiment of the present disclosure, the microchannel 310 has alignment grooves 320 on the inner side.

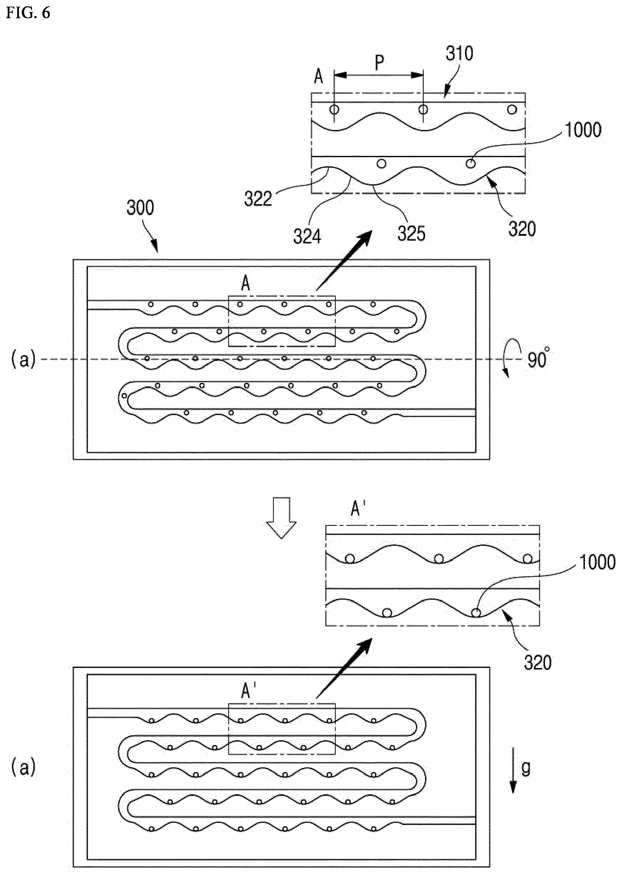

The alignment grooves 320 are described with reference to part A of FIG. 4 and FIG. 6A. The alignment grooves 320 are formed continuously along the inner side of the microchannel 310, so that the inner side extends in a wave shape. The pitch of the alignment groove 320 is defined as P. According to an embodiment of the present disclosure, the alignment grooves 320 may be formed as a sine curve having a period (pitch) of 3 to 5 mm.

The alignment grooves 320 have an inclined surface 324 extending from an upper part 322 toward a lower part 325. When the lower part 325 of the alignment grooves 320 forms a lower surface in the direction of gravity, the organoid seeds 1000 positioned on the inner side of the microchannel 310 are seated by moving to the lower part 325 of the alignment groove 320 along the inclined surface 324. Accordingly, when the organoid seeds 1000 are separately supplied along the microchannel 310 in response to the pitch P of the alignment grooves 320, and then the microfluidic chip 300 is erected, the inner side of the microchannel 310 becomes a bottom surface in the direction of gravity, and the organoid seeds 1000 are aligned evenly at the lower part 325.

In this specification, a state in which the upper surface 303 and the lower surface 305 of the microfluidic chip 300 are disposed in a cross direction with respect to the direction of gravity is defined as a horizontal state, and a state in which the lower surface of the microchannel in the direction of gravity becomes the inner side where the alignment grooves 320 are formed is defined as an vertical state. In the drawing, the case where the microfluidic chip 300 is arranged in the horizontal state and the case where the microfluidic chip 300 is arranged in the vertical state are distinguished by a direction indicator (arrow) of applying the gravity (g) indicated along with the drawing.

The injection of the organoid seeds and the collection of the cultured organoids are performed in a horizontal state with the microfluidic chip 300 disposed in parallel, and the alignment, levitation, holding, and culturing of the organoid seeds are performed in the vertical state.

FIG. 6 is a diagram for describing an alignment process of organoid seeds in a device for three-dimensional levitation culture of organoids according to an embodiment of the present disclosure.

FIG. 6A is a plan view of a microfluidic chip in a horizontal state, in which the upper surface 303 of the microfluidic chip 300 is positioned in an upward direction. While the organoid seeds are injected into the microchannel 310 of the microfluidic chip 300, the organoid seeds are injected separately at intervals corresponding to the pitch P of the alignment grooves 320. After the organoid seeds are injected, the valves 332 and 334 are closed to stop the fluid flow within the microchannel 310, and then the microfluidic chip 300 turns around 90°.

FIG. 6B is a diagram viewed from the front in the erected state of the microfluidic chip 300. The upper surface 303 of the microfluidic chip 300 is positioned in a lateral direction corresponding to the front. In the state illustrated in FIG. 6B, the gravity acts in the direction of the alignment grooves 320, and the inner side of the microchannel 310 with the alignment grooves 320 becomes the bottom surface in the direction of gravity.

An enlarged view of part A of FIG. 6A and an enlarged view of part A' of FIG. 6B are compared with each other. When the inner side is rotated to be the lower surface in the direction of gravity while the fluid flow in the microchannel 310 is stopped, the organoid seeds 1000 descend in the direction of gravity and move to the lower part 325 of the alignment grooves 320 along the inclined surface 324 and then stay. That is, the organoid seeds are introduced into the alignment grooves 320 and then aligned at the lower part 325 of the alignment grooves 320.

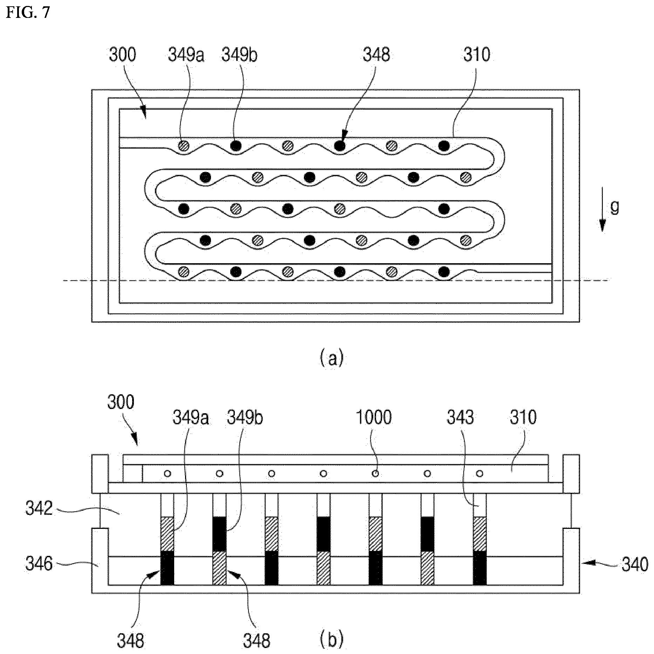

FIG. 7 is a diagram for describing coupling of a microfluidic chip and a magnet array according to an embodiment of the present disclosure, and FIG. 7A shows an arrangement relationship of the microfluidic chip and magnet poles of the magnet array, and FIG. 7B shows the magnet array arranged on one side of an erected microfluidic chip.

The magnet array 340 includes a plurality of magnets 348 arranged in response to the organoid seeds aligned within the microchannel 310. The magnet 348 provides magnetic force to levitate the organoid seeds 1000 within the microchannel 310 to a predetermined position and hold the organoid seeds in the levitated position.

According to an embodiment of the present disclosure, a permanent magnet may be used as the magnet 348. According to an embodiment of the present disclosure, the magnet 348 may be a cylindrical bar magnet having an aspect ratio of 5 to 10 and having magnetic poles arranged to face each other. In this specification, the bar magnet is used to refer to a permanent magnet with opposing magnetic poles arranged opposite to each other.

The bar magnet has two poles, that is, a N pole 349a and a S pole 349b, and the magnetic poles 349a and 349b of the bar magnet are arranged to match the levitation and holding positions of the organoid seeds within the microchannel 310. Since the position of the alignment grooves 320 within the microchannel 310 is specified, the placement position of the magnetic poles 349a and 349b may also be specified.

As shown in FIG. 7A, the adjacent magnetic poles 349a and 349b arranged on the same plane have different polarities. That is, the N pole 349a is disposed as a magnetic pole adjacent to the S pole 349b. While a magnetic force distribution acting on each organoid seed is formed independently when the polarities are disposed to cross each other, a cylindrical, concentrated magnetic force zone is formed at the position thereof. Therefore, when magnets are arranged in a form where adjacent magnetic poles cross each other, the position of each organoid seed may be precisely specified, and each organoid seed may be levitated independently. As a result, it is possible to prevent adjacent organoid seeds from agglomerating together.

Referring to FIG. 7B, the magnet array 340 includes a spacer 342, a magnet 348, and a magnet fixing part 346.

FIG. 7B illustrates that the magnet array 340 is arranged on the second surface 305, which is one surface of the microfluidic chip, but the magnet array 340 is arranged on each of both surfaces 303 and 305 of the microfluidic chip.

The spacer 342 has a plurality of magnet insertion grooves 343, and the position of each magnet insertion groove 343 is formed in response to the levitation and alignment positions of the organoid seeds in the microchannel 310 of the microfluidic chip 300.

The magnet 348 is inserted and fixed into the magnet insertion groove 343 of the spacer 342. A bar magnet is used as the magnet 348 so as to selectively control the magnetic force by stacking a desired number of magnets on the magnet insertion groove 343. For example, if the concentration of magnetic nanoparticles in the organoid seeds is set low as needed, levitation may be achieved by increasing the number of magnets to provide stronger magnetic force.

The magnet fixing part 346 fixes the magnet 348 so that a plurality of magnets 348 may be simultaneously coupled to or separated from the spacer 342.

A plurality of cylindrical bar magnets 348 inserted along the magnet insertion grooves 343 are coupled with the spacer 342 while being fixed vertically to the magnet fixing part 346.

In the magnet array 340, the spacer 342 and the magnet fixing part 346 having the magnets 348 are separately formed and may be coupled to the sides of the microfluidic chip 300, respectively. However, the magnet array 340 is not limited thereto and may be formed integrally or may be assembled first and then coupled to the side of the microfluidic chip 300.

FIG. 8 is a diagram showing a process of coupling magnet arrays 340 to both sides of a microfluidic chip. Magnetic poles 349a and 349b facing each other with the microfluidic chip 300 interposed therebetween have opposite polarities in the magnetic arrays 340 of the both sides.

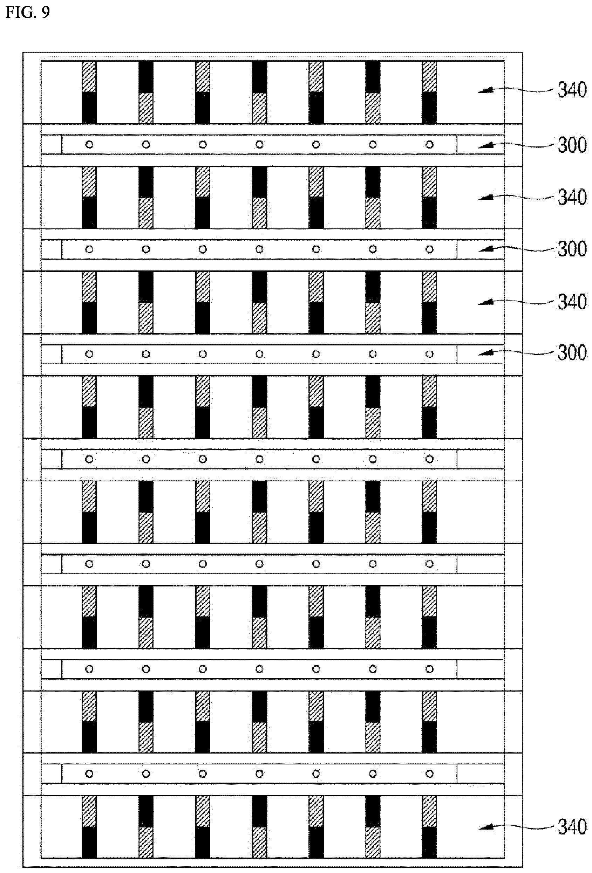

FIG. 9 illustrates a form in which a plurality of microfluidic chips is assembled in parallel in a device for three-dimensional levitation culture of organoids according to an embodiment of the present disclosure.

A magnet array 340 is disposed between microfluidic chips 300, and the magnet arrays 340 are disposed on both outmost sides. According to an embodiment of the present disclosure, it is possible to culture organoids by arranging and assembling the microfluidic chips 300 in parallel. This structure is advantageous for mass production of organoids.

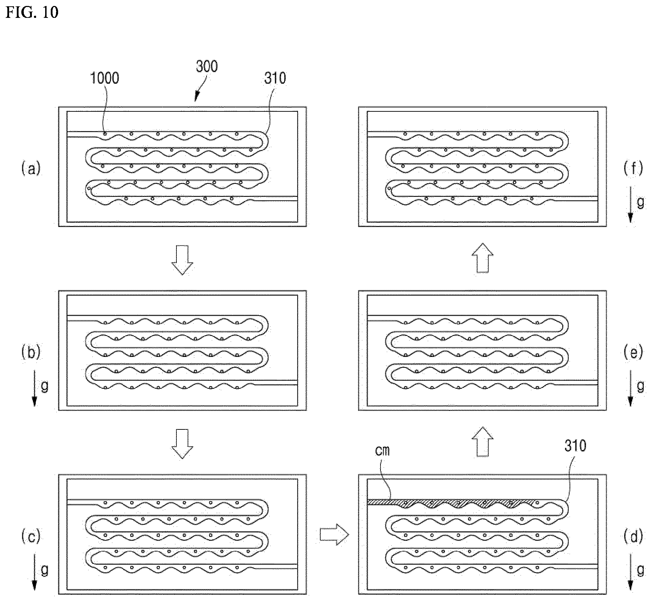

FIG. 10 is a diagram for sequentially describing coupling of a microfluidic chip and a magnetic array and a method for three-dimensional culture of organoids using the same.

As shown in FIG. 10A, organoid seeds are continuously supplied separately to the microchannel 310 of the microfluidic chip 300 in response to the pitch of the alignment grooves 320.

Referring to FIG. 10B, the valves are then closed to prevent the fluid movement within the microchannel and hold the organoid seeds, and then the microfluidic chip is rotated 90 degrees to induce and align the organoid seeds to the alignment grooves 320 using gravity.

Referring to FIG. 10C, thereafter, the organoid seeds are levitated upward in the direction of gravity with respect to the alignment grooves 320 using the magnets 348 to position the organoid seeds in the middle portion of the microchannel 310.

Referring to FIG. 10D, thereafter, while the organoid seeds are held in a levitating position by magnetic force, the organoid seeds are continuously cultured until a desired size is reached while the culture medium (cm) is replaced.

Referring to FIG. 10E, when the organoid has been cultured to a desired size, the magnet array 340 is separated. The organoids are aligned within the alignment grooves 320 by gravity.

Referring to FIG. 10F, the microfluidic chip 300 is rotated 180 degrees from the state of FIG. 10E. As a result, the cultured organoids are detached from the alignment grooves 320 and positioned on the flat inner side of the microchannel. In this state, the fluid is injected by opening the valves, and the organoids together with the fluid are discharged and collected through the outlet.

### Experimental Example

5 × 10⁷ human embryonic kidney (HEK) cells were dispersed in 10 µl of an organoid culture medium (ThermoFisher) and stained with 10 µl of trypan blue. Thereafter, after the supernatant was removed, organoid seeds were prepared using an organoid seed composition prepared by adding 5 ml of alginate and 30 µg/ml of magnetic nanoparticles (MNP, particle size 20 nm).

The organoid seed composition was injected into a microchannel to be formed in a droplet shape, and then a calcium chloride solution was injected into the microchannel to prepare gelled organoid seeds.

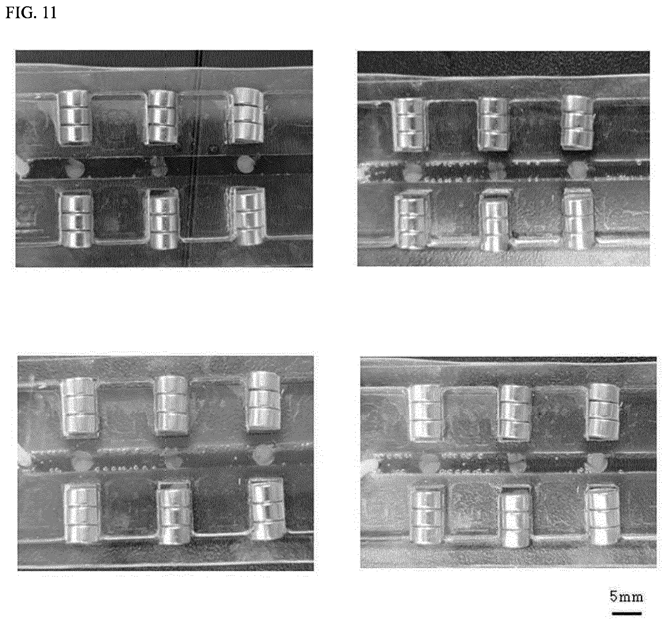

FIG. 11 is a diagram showing a process of exchanging a medium after injecting organoid seeds into a microchannel of a microfluidic chip and levitating the organoid seeds by magnetic force. Organoid seeds with a diameter of approximately 2 mm were injected into the microchannel. As shown in FIG. 11, it may be confirmed that the culture is performed when the organoid seeds are levitated by magnetic force even during the process of replacing the culture medium. It was confirmed that air bubbles were generated within the culture medium, but did not interfere with medium exchange.

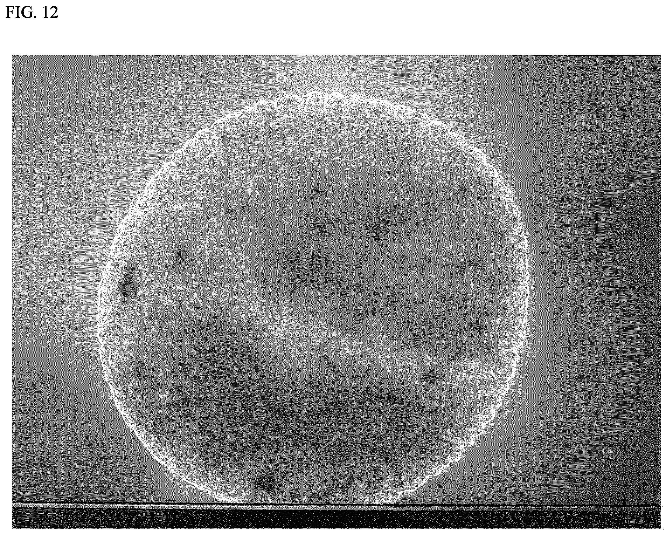

FIG. 12 is an image of HEK cells cultured in a microchannel of a microfluidic chip while being levitated by magnetic force and replacing an organoid culture medium (ThermoFisher, 11965-DMEM, high glucose) three times for 72 hours. It was confirmed that the culture was performed while maintaining a three-dimensional shape.

## Claims

1. A method for three-dimensional levitation culture of organoids comprising:
a) injecting a fluid including organoid seeds, which include magnetic nanoparticles, into a microchannel of a microfluidic chip;
b) providing magnetic force formed by means of a magnet to the organoid seeds injected into the microchannel to levitate the organoid seeds at a culture position;
c) holding the organoid seeds in the culture position by means of the magnetic force;
d) culturing the organoid seeds into an organoid while replacing a culture medium in the microchannel; and
e) collecting the cultured organoids.

2. The method for three-dimensional levitation culture of organoids of claim 1, wherein the organoid seeds include stem cells, growth factors, and biodegradable materials.

3. The method for three-dimensional levitation culture of organoids of claim 2, wherein the organoid seeds are in a gelled state.

4. The method for three-dimensional levitation culture of organoids of claim 1, further comprising:
an alignment step of aligning each of the organoid seeds at positions set to be spaced apart from each other before step b),
wherein the alignment step is performed by capturing the organoid seeds within alignment grooves formed in the microchannel while blocking the inflow and outflow of the fluid in the microchannel.

5. The method for three-dimensional levitation culture of organoids of claim 4, wherein the alignment grooves are formed to be continuously connected to each other along the inner side of the microchannel so that the connected form of the alignment grooves has a wave shape,
the alignment grooves are formed to have constant pitches, the organoid seeds are injected so as to be spaced apart from each other in response to the pitches of the alignment grooves, and
the organoid seeds are aligned by moving to a lower part along the inclined surface of the alignment grooves by gravity.

6. The method for three-dimensional levitation culture of organoids of claim 5, wherein the alignment grooves are formed along the inner side of the microchannel, and
the alignment step is performed by rotating the microfluidic chip so that the alignment grooves face downward.

7. The method for three-dimensional levitation culture of organoids of claim 1, wherein the magnetic poles of the magnets facing each other with the microfluidic chip interposed therebetween have different polarities.

8. The method for three-dimensional levitation culture of organoids of claim 7, wherein the magnetic poles of the adjacent magnets arranged on the same plane have different polarities.

9. A device for three-dimensional levitation culture of organoids comprising: an inlet into which a fluid including organoid seeds, which include magnetic nanoparticles, and a culture medium are injected; a microchannel connected to the inlet and into which the organoid seeds are separately injected at predetermined injection intervals and then moved, and held and cultured at a predetermined culture position; an outlet through which the culture medium and the cultured organoids are discharged; a microfluidic chip including valves capable of blocking the movement of the fluid to the inlet and the outlet; and
a magnet array disposed on each of both opposite surfaces of the microfluidic chip, and including magnets providing magnetic force to levitate and hold the organoid seeds in the microchannel at the culture position.

10. The device for three-dimensional levitation culture of organoids of claim 9, wherein alignment grooves are formed on the inner surface of the microchannel so that the organoid seeds move downward by gravity and are aligned.

11. The device for three-dimensional levitation culture of organoids of claim 10, wherein the alignment grooves are formed along the inner side of the inner surfaces of the microchannel, and the organoid seeds are aligned by rotating the microfluidic chip so that the alignment grooves face downward.

12. The device for three-dimensional levitation culture of organoids of claim 11, wherein the alignment grooves are formed to be continuously connected to each other along the inner side so that the inner side has a wave shape,
the alignment grooves are formed to have constant pitches, and the organoid seeds are injected to be spaced apart from each other in response to the pitches of the alignment grooves.

13. The device for three-dimensional levitation culture of organoids of claim 9, wherein the magnet array comprises
a spacer having a plurality of magnet insertion grooves into which the magnets are inserted, and
a plurality of magnets inserted into the magnet insertion grooves,
wherein when the magnet arrays are arranged on both surfaces of the microfluidic chip, respectively, the magnetic poles of the magnets are aligned in response to the culture position of the organoid within the microchannel.

14. The device for three-dimensional levitation culture of organoids of claim 13, wherein in the magnet array, the magnetic poles of the magnets facing each other with the microfluidic chip interposed therebetween have different polarities.

15. The device for three-dimensional levitation culture of organoids of claim 13, wherein in the magnet array, the magnetic poles of the adjacent magnets arranged on the same plane have different polarities.
